(19) Europäisches Patentamt
European Patent Office
Office européen des brevets

(11) Veröffentlichungsnummer: **0 459 284 B1**

## (12) EUROPÄISCHE PATENTSCHRIFT

(45) Veröffentlichungstag der Patentschrift: **25.01.95**

(51) Int. Cl.$^6$: **A61M 16/04**

(21) Anmeldenummer: **91108242.8**

(22) Anmeldetag: **22.05.91**

(54) **Vorrichtung zur automatischen Steuerung der Spontanatmungsunterstützung.**

(30) Priorität: **22.05.90 DE 4016401**

(43) Veröffentlichungstag der Anmeldung:
**04.12.91 Patentblatt 91/49**

(45) Bekanntmachung des Hinweises auf die
Patenterteilung:
**25.01.95 Patentblatt 95/04**

(84) Benannte Vertragsstaaten:
**CH DE FR GB LI SE**

(56) Entgegenhaltungen:
EP-A- 0 074 809    EP-A- 0 092 618
EP-A- 0 147 516    EP-A- 0 366 524
DE-A- 3 204 110    US-A- 3 841 319

**Gärtner: Beatmungs- und Narkosetechniken**

(73) Patentinhaber: **Obermayer, Anton, Dr.-Ing.**
**Rudelsweiherstrasse, 15A**
**D-91054 Erlangen (DE)**

(72) Erfinder: **Obermayer, Anton, Dr.-Ing.**
**Rudelsweiherstrasse, 15A**
**D-91054 Erlangen (DE)**

(74) Vertreter: **Patentanwälte Dipl.-Ing. E. Eisele**
**Dr.-Ing. H. Otten**
**Seestrasse 42**
**D-88214 Ravensburg (DE)**

**Beschreibung**

Die Erfindung betrifft eine Vorrichtung zur automatischen Steuerung der Spontanatmungsunterstützung nach dem Oberbegriff des Anspruchs 1.

Problemstellung und Stand der Technik:

Nach großen chirurgischen Eingriffen, bei traumatisch hervorgerufenen oder durch bestimmte Grunderkrankungen bedingten Beeinträchtigungen der Lungenventilation werden die Patienten im allgemeinen einer sogenannten Respiratortherapie unterzogen. Der erste Abschnitt dieser Behandlung umfaßt die maschinelle Beatmung, die ausschließlich durch die geräteseitigen Einstellungen am Respirator bestimmt ist und somit ohne aktive Beteiligung des Patienten ausgeführt wird. Der zweite Abschnitt der Respiratortherapie dient der nach längeren Beatmungszeiten erforderlichen Umstellung der Ventilation von der Beatmung auf die Spontanatmung (= Eigenatmung) des Patienten, wozu heute eine ganze Reihe von Verfahrensschritten (Weaningverfahren) zur Verfügung stehen.

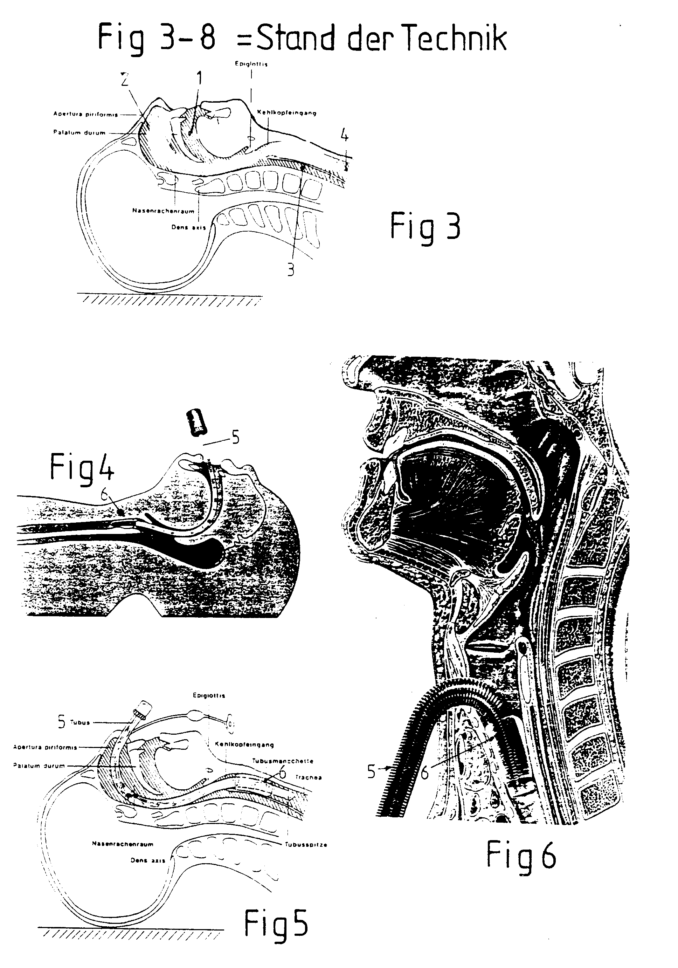

Nachfolgend wird der Stand der Technik anhand der Figuren 3 bis 8 näher erläutert (siehe Lit. Gärtner: "Beatmungs- und Narkosetechniken).

Das zentrale Problem aller Weaningverfahren besteht darin, daß der Patient bzw. die Atemwege des Patienten, wie in Fig. 3 näher dargestellt, luftdicht mit dem Respirator verbunden werden müssen. In Fig. 3 ist hierfür mit Bezugszeichen 1 der Mundraum, mit Bezugszeichen 2 der Nasenraum und mit Bezugszeichen 3 die Speiseröhre gezeigt. Die Trachea ist mit Bezugszeichen 4 gezeigt.

Zur Verbindung der Atemwege mit dem Respirator werden üblicherweise eine orale (Fig. 4) oder eine nasale (Fig. 5) Intubation oder gemäß der Darstellung in Fig. 6 eine Tracheotomie, d. h. ein unmittelbarer Eingang in die Trachea, eingesetzt. Die Wirkungsweisen dieser drei Verfahren sind - wie dies auch aus den Figuren 3 bis 6 hervorgeht - im Prinzip die gleichen, da in die zur Verfügung stehenden großvolumigen Atemwege mit Trachealdurchmesser von 2 bis 2,5 cm ein sogenannter Endotrachealtubus 5 mit einem Durchmesser von etwa 0,6 bis 0,8 cm eingesetzt wird. Am unteren Ende des Endotrachealtubus 5 befindet sich ein aufblasbarer Ballon 6, der zur Abdichtung der unteren Atemwege gegenüber der Atmosphäre dient. Dies schützt den Patienten einerseits gegen das äußerst gefährliche Eindringen von Magensaft in die Atemwege und verhindert gleichzeitig unerwünschte Gasverluste während der Respiratortherapie.

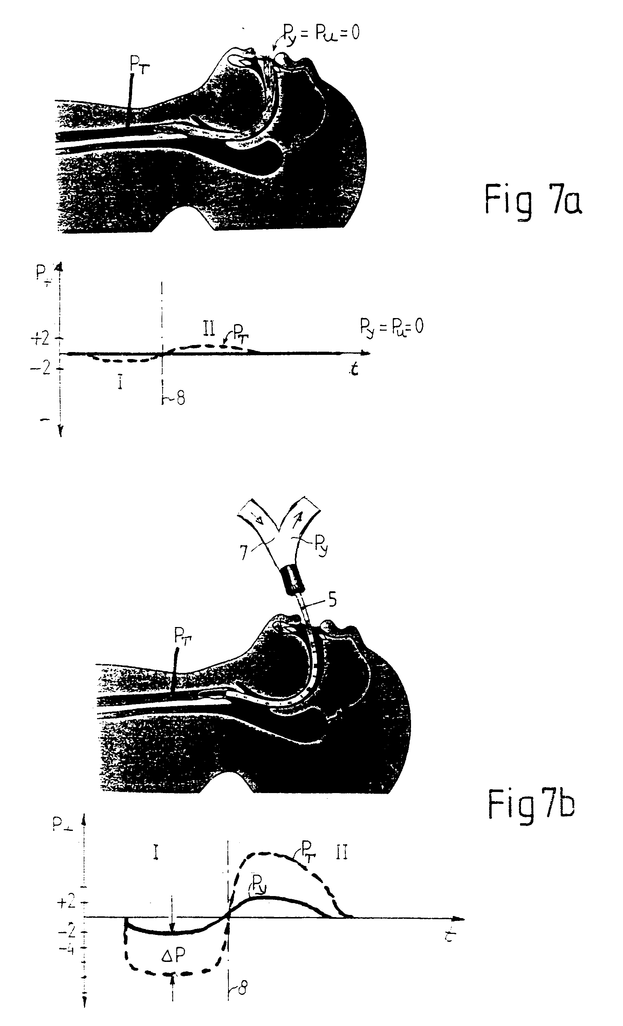

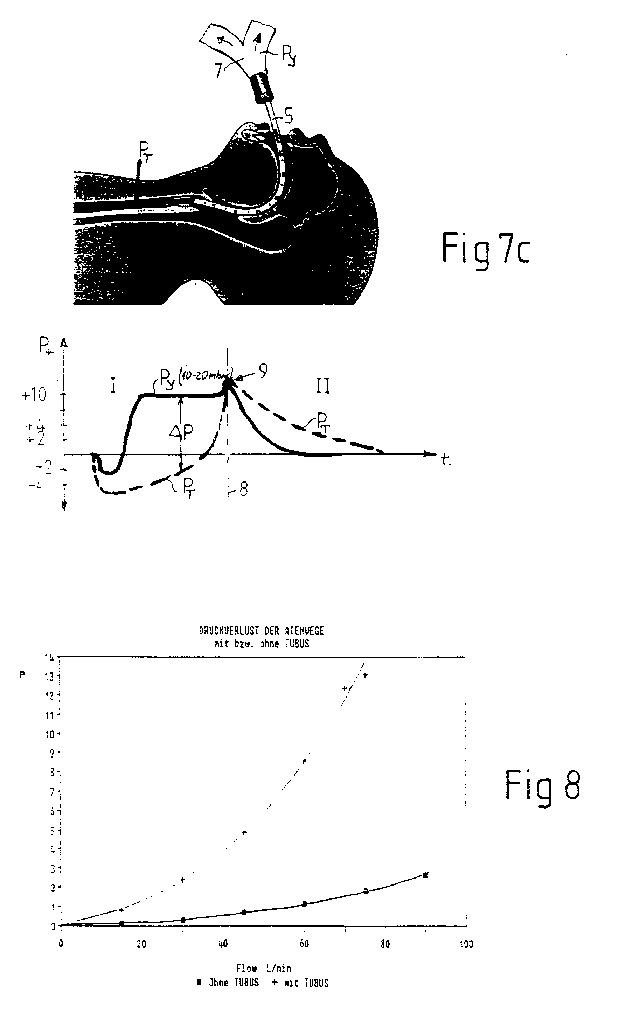

Diesem Vorteil des Endotrachealtubus 5 steht als Nachteil der gegenüber den physiologischen Atemwegen gemäß Fig. 3 erheblich höhere Reibungsdruckverlust gegenüber. Dieser Reibungsdruckverlust ist für verschiedene Atmungen in den Figuren 7a bis 7c sowie in Fig. 8 als Diagramm dargestellt. Dabei zeigt Fig. 7a den Druckverlauf bei der Spontanatmung ohne Tubus und Fig. 7b den Druckverlauf bei der Spontanatmung mit Endotrachealtubus 5. Dabei ist unterhalb der jeweiligen Figurendarstellung ein Druck-Zeit-Diagramm (P/t-Diagramm) dargestellt, mit der Einatmungsphase I unterhalb der Zeit-Achse t und der Ausatmungsphase II oberhalb der Zeit-Achse t. Mit $P_U$ ist der Umgebungsdruck, mit $P_Y$ der Druck im Y-Stück zum Respirator gekennzeichnet. $P_T$ (gestrichelte Kurve) gibt den intratrachealen Druck an, der gemäß der Kurvendarstellung um ca. +/- 2 mbar schwankt. Die Einatmungsphase I und die Ausatmungsphase II wird durch die Linie 8 getrennt.

Diese Druckverhältnisse ändern sich drastisch bei der Darstellung des Druckverlaufs bei der Spontanatmung mit einem Tubus gemäß Fig. 7b. Hier ist ein Überdruck bzw. Unterdruck im Y-Stück 7 vorgesehen, der mit $P_Y$ gekennzeichnet ist. Der intratracheale Druck $P_T$ ist in der graphischen Darstellung gestrichelt, der Druck im Y-Stück als durchgezeichnete Linie in der Einatmungsphase I und in der Ausatmungsphase II eingezeichnet Dabei ist die Druckdifferenz $\Delta P$ flußabhängig, wie dies aus Fig. 8 erkennbar ist. Grundsätzlich ist die Druckdifferenz $\Delta P$ mit Tubus wesentlich größer als ohne einen Tubus gemäß Darstellung in Fig. 7a.

Aus den vorstehenden Erläuterungen ergibt sich, daß dem durch die Grunderkrankung ohnehin geschwächten Patienten eine wesentlich erhöhte Atemleistung abverlangt wird. Da der Patient jedoch häufig nicht in der Lage ist, diese vermehrte Atemleistung zu erbringen, führt die derzeitige Durchführung des Weanings zu unvermeidbaren Belastungen des Patienten.

Eine Verbesserung dieses Weaningverfahrens wurde gegen Ende der 70er Jahre durch die sogenannte assistierte oder druckunterstützte Spontanatmung in die Respiratortherapie eingefügt. Dies ist näher in Fig. 7c dargestellt. Diese Figur zeigt den Druckverlauf bei der assistierten Spontanatmung, wobei wiederum mit $P_Y$ der Druck im Y-Stück 7 und mit $P_T$ der Intratrachealdruck gezeigt ist. Die Trennlinie zwischen Einatmungsphase I und Ausatmungsphase II ist mit Bezugszeichen 8 gekennzeichnet.

Das Prinzip dieser in Fig. 7c dargestellten Spontanatmungsform besteht darin, daß der Patient durch eine spontane Einatmung (Inspiration) einen Unterdruck unter das vorgegebene endexspiratorische Druckniveau erzeugt. Dieser wird durch die sogenannte Triggereinrichtung (Schwellwertschalter) erfaßt und löst - über den Schwellwertschalter - im Respirator eine Gaslieferung in der Form aus, daß am Y-Stück 7 der durch den Anwender fest eingestellte Unterstützungsdruck $P_Y$ bis zum Umschalten auf Ausatmung (Exspirationsphase II) aufrechterhalten wird. $P_Y$ kann hierbei einen Wert von 10 bis 20 mbar einnehmen. Mit Bezugzeichen 9 ist der Umschaltpunkt zwischen Einatmungs- und Ausatmungsphase gezeigt.

Trotz erheblicher Verbesserung gegenüber früheren Verfahren weist jedoch die assistierte Spontanatmung nach wie vor folgende erhebliche Nachteile auf:

1. Zur Erzeugung des Unterdrucks, der über die Triggereinrichtung den Aufbau des fest eingestellten Unterdrucks auslöst, muß der Patient unter Überwindung des Reibungsdruckverlustes des Endotrachealtubus evakuieren, wodurch ein unverzügliches Ansprechen der Triggereinrichtung verhindert wird.

2. Der Aufbau des Unterstützungsdrucks erfolgt nach der Antriggerung bis zum fest eingestellten Druckwert am Y-Stück 7, wodurch die eigentlich notwendige Anpassung an die vom jeweiligen Gasfluß abhängigen Druckverluste (siehe hierzu Fig. 8) nicht beachtet wird. Dies führt in der Regel dazu, daß abhängig von dem durch die Einatmung des Patienten erzeugten Gasflusses abwechselnd eine Über- oder Unterkompensation des im Endotrachealtubus auftretenden Druckverlustes hingenommen werden muß.

3. Durch den Aufbau des positiven Unterstützungsdrucks am Y-Stück 7 des Respirators kommt es in jedem Fall - auch ohne eine nach der Druckauslösung fortbestehende Eigenatmung - zu einem Gasfluß in die Lunge des Patienten bis hin zu einem Druckausgleich zwischen dem positiven Atemwegsdruck am Y-Stück 7 und dem in der Lunge. Dieser Gasabfluß bzw. Druckausgleich entspricht im Prinzip aber einer durch den Patienten ausgelösten Beatmung und steht im krassen Widerspruch zur eigentlichen Zweckbestimmung der assistierten Spontanatmung, nämlich der Mithilfe des Respirators bei Eigenatmung des Patienten.

4. Ein weiteres Problem der herkömmlichen Ausführung der assistierten Spontanatmung besteht in der Festlegung des Umschaltkriteriums von der Inspiration auf die Exspiration, d. h. wie erkennt der Respirator den Beginn der spontanen Ausatmung durch den Patienten. Üblicherweise wird dieses Umschaltkriterium so festgelegt, daß der Patient durch seine Ausatmung den Druck im gesamten System (Atemwege und Schlauchsystem) so anheben muß, daß der Atemwegsdruck das Umschaltkriterium, nämlich den eingestellten Unterstützungsdruck + 1 bis 2 mbar, überschreitet, wodurch der Respirator dann auf Exspiration umgeschaltet wird. Für den Patienten ergibt sich hieraus eine Verzögerung und eine Erschwerung der Ausatmungsphase.

Aus der Druckschrift DE-A-3 204 110 ist zwar ebenfalls ein Trachialtubus bekanntgeworden, der einen Beatmungsschlauch sowie eine Druckmeßkanüle zur intratrachialen Druckmessung umfaßt. Diese Vorrichtung wird jedoch zur künstlichen Beatmung bei der Chirurgie eingesetzt, wobei die Erfassung des intratrachialen Druckes der Regelung der Zufuhr des Atemgases bei der künstlichen Beatmung dient und wobei insbesondere ein überhöhter Lungendruck vermieden werden soll. Bei durch die Druckmessung erkannten Störungen, zum Beispiel eine auftretende Spontanatmung des Patienten, muß zur Reduzierung eines überhöhten Lungendruckes ein Ballon zur Sicherheit entlastet werden.

Die bekannte Vorrichtung arbeitet demnach mit einer Beatmungsmaschine bei einem völlig bewußtlosen Patienten, der nach einem voreingestellten Muster beatmet wird. Sie betrifft nicht die Spontanatmungsunterstützung mit einer entsprechenden automatischen Regelung, wie dies in der eingangs gewürdigten Literatur beschrieben ist.

Vorteile der Erfindung:

Das Ziel jeder Verbesserung der Atemgaslieferung für den an einem Respirator spontan atmenden Patienten muß daher die Vermeidung der vorgenannten Nachteile, nämlich

- verzögertes Ansprechverhalten der Triggereinrichtung
- nicht an den Gasfluß adaptierter Druckaufbau
- Möglichkeit des unbemerkten Übergangs zur Beatmung und
- verspätete Umschaltung auf Exspiration

sein. Diese Nachteile werden durch die vorliegende Erfindung beseitigt.

Zur Lösung der geschilderten Problematik bietet sich an, den Atemwegsdruck - unter Umgehung der hochdynamischen Strömungsvorgänge im Endotrachealtubus - direkt in den unteren Atemwegen über eine geeignete Druckmeßvorrichtung zu erfassen und diese zur Steuerung bzw. zur Regelung der Gaslieferung und der Druckunterstützung durch den Respirator zu verwenden.

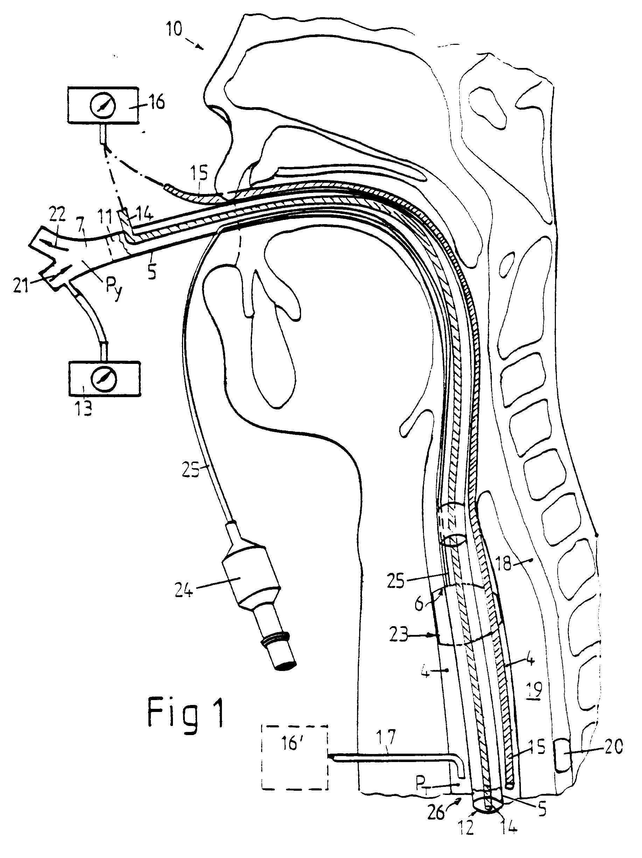

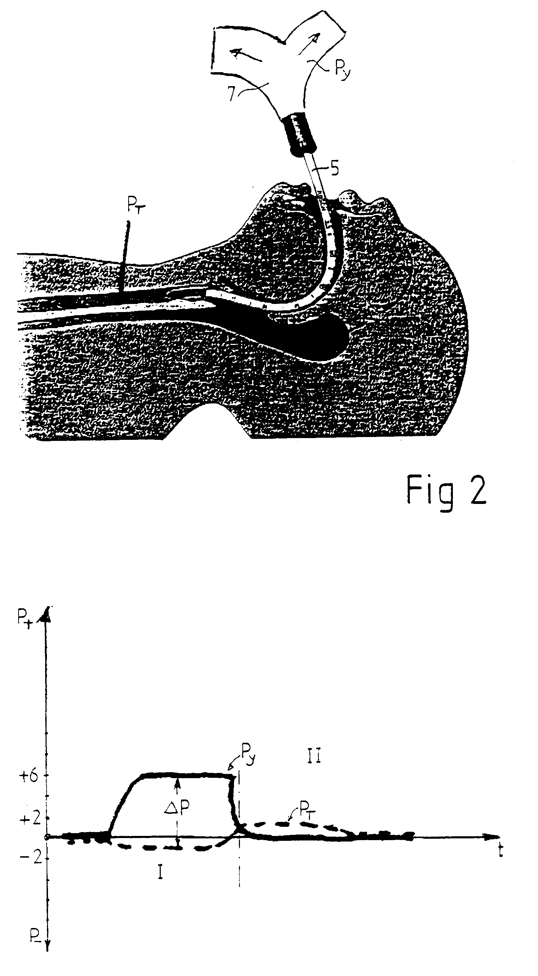

In den Figuren 1 und 2 wird eine solche Anordnung dargestellt. In der nachfolgenden Beschreibung wird eine solche Anordnung näher erläutert.

Fig. 1     zeigt eine Anordnung der intertrachealen Druckmessung,

Fig. 2     den Druckverlauf bei der Spontanatmung mit automatisierter Druckunterstützung gemäß Vorgang in Fig. 1.

In Fig. 1 ist mit Bezugszeichen 10 der Kopf des Patienten gezeigt, an welchem eine orale Intubation vorgenommen wird. Über das Y-Stück 7 ist ein nicht näher dargestellter Respirator an einen Endotrachealtubus 5 angeschlossen. Der Tubus 5 weist im unteren Trachealbereich 23 einen über die Leitung 25 mittels des Ansatzstückes 24 aufblasbaren Ballon 6 auf, der den Tubus 5 gegenüber der Trachea 4 abdichtet. Der Tubuseingang ist mit Bezugszeichen 11 gekennzeichnet. Das Ende des Tubus 5 im unteren Bereich 26 der Trachea 4 ist mit Bezugszeichen 12 gezeigt. Eine Druckmeßeinrichtung 13 am Y-Stück 7 bzw. am Respirator dient zur Erfassung des Drucks $P_Y$.

Zusätzlich zum Endotrachealtubus 5 sind eine oder zwei Druckmeßleitungen 14, 15 vorgesehen, die sich beispielsweise als Druckmeßleitung 14 innerhalb des Tubus 5 und/oder als Druckmeßleitung 15 außerhalb des Tubus 5 befinden. Die Druckmeßleitungen 14, 15 sind mit einem Druckmeßgerät 16 verbunden, welches den Trachealdruck im unteren Teil 26 der Trachea 4 mißt. Mit Bezugszeichen 17 kann eine weitere oder eine alternative Druckmeßleitung vorgesehen sein, die aufgrund einer Tracheotomie, d. h. unmittelbar von außen, in die Trachea 4 zur Druckerfassung eingebracht ist. Mit Bezugszeichen 18 ist die Speiseröhre, mit Bezugszeichen 19 die Tracheawand in Fig. 1 dargestellt. Eine zusätzliche Meßvorrichtung 20 (Ösophagussonde) ist in der Speiseröhre 18 angeordnet. Mit Bezugszeichen 21 ist der Gasfluß vom Respirator, mit Bezugszeichen 22 der Gasfluß zum Respirator gekennzeichnet.

Wird der intratracheale Atemwegsdruck $P_T$ - wie in Fig. 1 dargestellt - mittels einer geeigneten Meßeinrichtung 16, 16' unterhalb der Tubusspitze 12 gemessen, so ergibt sich als Vorteil eine im Vergleich zur bisherigen Druckmessung im Respirator wesentliche Erhöhung der Ansprechgeschwindigkeit der Triggereinrichtung im Respirator. Dies ist in Fig. 2 im Kurvendiagramm dargestellt. Der Patient muß zwar in prinzipiell gleicher Weise einen Unterdruck unter das endexspiratorische Druckniveau zur Auslösung der Gaslieferung durch den Respirator erzeugen. Der wesentliche Unterschied zur bekannten Druckmessung besteht aber darin, daß dieser Unterdruck lediglich im unteren Teil 26 der Trachea 4 erreicht werden muß. Dies bedeutet, daß die ansonsten notwendige Evakuierung des Schlauchsystems zwischen dem Y-Stück 7 und dem Respirator und die Erzeugung des dynamischen Druckabfalls längs des Endotrachealtubus 5 entfällt. Als Vorteile für den Patienten ergeben sich hieraus eine schnellere Auslösung der Gaslieferung und eine Verringerung der Atemleistung.

Ein weiterer Vorteil der vorgeschlagenen Meßeinrichtung besteht darin, daß am Y-Stück 7 ein Atemwegsdruck aufgebaut wird, der den flußabhängigen Reibungsdruckverlust (siehe Fig. 8) des Endotrachealtubus 5 kompensiert, d. h. die Höhe der Druckunterstützung wird Atemzug für Atemzug an den jeweils vom Patienten erzeugten Gasfluß angepaßt. Hierdurch entfällt der ständige Wechsel zwischen der Über- und Unterkompensation, wie er bei der bisherigen starren Einstellung der Höhe des Unterstützungsdruckes hingenommen werden muß. Die flußabhängige Kompensation beruht darauf, daß der Respirator versucht, das am Respirator vorgewählte intratracheale Druckniveau während einer spontanen Inspiration aufrechtzuerhalten. Regeltechnisch gesehen, gelingt die Aufrechterhaltung des Intratrachealdrucks aber nur dann, wenn der Respirator über den Tubus die Gasmenge liefert, die der Patient durch seine Spontanatmung aus der Trachea in die Lunge hinein abzieht. Die Lieferung der geforderten Gasmenge bei vorgegebenem Intratrachealdruck gelingt dem Respirator wiederum nur, wenn am Y-Stück ein Überdruck aufgebaut wird, der dem flußbedingten Reibungsdruckverlust des Tubus entspricht.

Die geschilderte Regelung der Gaslieferung bei vorgegebenem Intratrachealdruck hat den weiteren Vorteil, daß der Überdruckaufbau am Y-Stück 7 nur dann stattfindet, wenn der Patient eine aktive Spontanatmung zeigt. Hierdurch wird im Gegensatz zur bekannten Ausführung der assistierten Spontanatmung, wo der Unterstützungsdruck auf jeden Fall aufgebaut wird, der unbemerkte Übergang von der assistierten Spontanatmung auf eine Beatmung ohne Patientenbeteiligung verhindert.

Eine weitere Verbesserung ergibt sich dadurch, daß bei der hier vorgeschlagenen Meßeinrichtung ein anderes Kriterium für die Umsteuerung von der Inspiration auf die Exspiration verwendet werden kann. Bei der bekannten Ausführung muß der Patient durch seine Ausatmung am Y-Stück einen Druck erzeugen, der um 1 bis 2 mbar über dem eingestellten Unterstützungsdruck liegt. Da dieser Druck entgegen dem Reibungsdruckverlust des Tubus erzeugt werden muß, erfordert das bislang eingesetzte Umschaltkriterium weit überhöhte intratracheale Atemwegsdrucke. Bei der hier vorgeschlagenen Regelung wird als Umschaltkriterium das gewünschte endexspiratorische Druckniveau plus 1 bis 2 mbar herangezogen, so daß eine geringfügige Ausatmung des Patienten auch eine sofortige Umschaltung des Respirators zur Folge hat. Das Verhältnis der vom Patienten zu erzeugenden Umschaltdrucke beträgt bei Standardeinstellung der konstanten Druckunterstützung etwa 1/10 bis 1/15. Für den Patienten ergeben sich aus den vorgeschlagenen Umsteuerkriterien in Verbindung mit der intratrachealen Druckmessung die Vorteile, einer weniger anstrengenden und schnelleren Umschaltung des Respirators von Inspiration auf Exspiration.

Da die Meßleitung 14 bis 17 nach dem Einsetzen in die Trachea 4 einer Beobachtung und einer einfachen Funktionskontrolle entzogen ist, muß die gesamte Meßkette bzw. Meßeinrichtung so ausgeführt werden, daß im Fehlerfall, etwa bei Verstopfung durch Blut und/oder Sekret oder bei Abknickungen der Meßleitung, eine sichere Funktion der Gaslieferung an den Patienten dennoch gewährleistet ist. Als mögliche Sicherheitseinrichtungen, die sowohl einzeln als auch in ihrer Kombination angewendet werden können, bieten sich an:

- eine zusätzliche Druckmeßeinrichtung 13 am Y-Stück
- eine indirekte Messung des Intratrachealdruckkes über die sogenannte Ösophagussonde 20 und
- eine kontinuierliche oder diskontinuierliche Spülung der Druckmeßleitung 14, 15.

Dies wird wie folgt beschrieben:

Eine sichere Erfassung des einwandfreien Funktionszustandes der intratrachealen Druckmeßeinrichtung gelingt insbesondere über eine Messung des Druckes am Y-Stück mittels der Meßeinrichtung 13. Dadurch, daß bei nicht durchströmtem Tubus die am Y-Stück 7 und intratracheal gemessenen Drücke identisch sein müssen, wogegen bei vorhandener Gasströmung durch den Tubus charakteristische Differenzen zwischen den beiden Druckmeßstellen 11 und 12 auftreten, die zusätzlich erfaßt und ausgewertet werden können.

Als weitere Möglichkeit bietet sich die Verwendung einer sogenannten Ösophagussonde 20 an, da die hintere elastische Trachealwand 19 und die ebenfalls elastische Speiseröhre 18 die intratrachealen Druckschwankungen auf den Ballon der Ösophagussonde 20 gut übertragen und dadurch indirekt meßbar machen. Die Validierung der direkt gemessenen intratrachealen Drücke erfolgt dann durch Vergleich mit den gemessenen Ösophagusdrucken und unter Umständen mit den am Y-Stück 7 ermittelten Druckwerten.

Die Aufrechterhaltung bzw. die Wiederherstellung einer einwandfreien Funktion der Meßleitung 14, 15 gelingt auch durch eine kontinuierliche oder diskontinuierliche Spülung der Meßleitung. Bei Anwendung einer kontinuierlichen Spülung kann die Verfälschung der intratracheal gemessenen Druckwerte durch eine Kalibrierung egalisiert werden.

Die in Fig. 2 dargestellte Anordnung der oralen Intubation mit einer Meß- und Regeleinrichtung, wie sie in Fig. 1 dargestellt und beschrieben ist, zeigt im unteren Bild einen Druckverlauf bei der Spontanatmung mit automatisierter Druckunterstützung, d. h. insbesondere mit der intratrachealen Druckmessung zur Kompensierung der Druckverluste. Dabei wird die Druckdifferenz $\Delta P$ zwischen dem in dem Diagramm dargestellten Druck im Y-Stück $P_Y$ und dem Druck in der Trachea $P_T$ aufgrund der intratrachealen Druckmeßeinrichtung gasflußabhängig geregelt. Der Verlauf des Intratrachealdrucks $P_T$ entspricht aufgrund der Regelung nahezu demjenigen, wie er in der Abbildung 7a für einen Druckverlauf bei der Spontanatmung ohne jeglichen Tubus gezeigt ist. Dabei schwankt der Intratrachealdruck in der Größenordnung von ca. +/- 2 mbar. Der Patient kann demnach in seiner Spontanatmung derart verfahren, als wenn kein Tubus vorhanden wäre. Insbesondere zeigt die Kurvendarstellung gemäß Fig. 2 nach der Erfindung eine deutliche Verbesserung gegenüber der Kurvendarstellung in Fig. 7b nach dem Stand der Technik.

Mit der erfindungsgemäßen Einrichtung wird demnach der eigentlich interessierende intratracheale Atemwegsdruck direkt ermittelt und zur Regelung der Gaslieferung des Patienten herangezogen.

Die Erfindung ist nicht auf das dargestellte und beschriebene Ausführungsbeispiel beschränkt. Sie umfaßt auch vielmehr alle fachmännischen Weiterbildungen im Rahmen der Ansprüche.

**Patentansprüche**

1. Vorrichtung zur automatischen Regelung der Spontanatmungsunterstützung am Respirator einer Beatmungsmaschine, mit einem Endotrachealtubus (5) mit eingangsseitigem, als Y-Stück (7) ausgebildeten Adapterstück zum Anschluß einer Inspirations- (21) bzw. Exspirationsleitung (22) an einen Respirator, wobei der Tubus (5) bis in die untere Hälfte der Trachea (4) reicht und wobei in der Inspirationsphase eine Atemwegsdruckmessung zur Regelung der Gaslieferung des Respirators vorgesehen ist und wobei weiterhin zur Kompensation der Reibungsdruckverluste des Gases im Tubus (5) ein vom Respirator erzeugter Überdruck $P_Y$ am Y-Stück (7) erzeugt wird, dadurch gekennzeichnet, daß zur automatischen Kompensation des Reibungsdruckverlustes des Endotrachealtubus (5) im unteren Trachealbereich (26) eine Druckmeßleitung (14, 15, 17) endet, die nach außen geführt ist und zur Messung des intratrachealen Atemwegsdruckes $P_T$ mit einem Druckmeßgerät (16) dient, daß der erforderliche Überdruck $P_Y$ am Y-Stück (7) in Abhängigkeit des vom Patienten bei seiner Spontanatmung erzeugten Gasflusses bzw. vom gewünschten oder erforderlichen Lungendruckniveau mittels des Respirators derart einstellbar ist, daß der am Tubusende (12) d.h. an der intratrachealen Druckmeßstelle gewünschte Atemwegsdruck $P_T$ sich einstellt, wobei bei fehlender Spontanatmung des Patienten kein Überdruck im Y-Stück erzeugt wird, daß im Tubuseingangsbereich (11) bzw. im Respirator eine Druckmeßeinrichtung (13) des dortigen Atemwegsdruckes $P_Y$ vorgesehen ist, die über eine Differenzschaltung mit der Trachealdruckmeßeinrichtung (16) verbunden ist und daß die während der einzelnen Atmungs- und Beatmungsphasen definierte Druckdifferenz $\Delta P = P_Y - P_T$ bzw. $P_T - P_Y$ erfaßbar ist, wobei Fehlfunktionen, ausgelöst durch Verstopfung, Abknickung oder Verschluß erfaßbar sind.

2. Vorrichtung nach Anspruch 1, dadurch gekennzeichnet, daß der am Tubusende (12) gemessene Atemwegsdruck die Gaslieferung über eine Triggereinrichtung (z.B Schwellwertschaltung) auslöst.

3. Vorrichtung nach Anspruch 1 oder 2, dadurch gekennzeichnet, daß die Atemwegsdruckmessung eine an den Patienten angepaßte Gaslieferung über den Respirator auslöst.

4. Vorrichtung nach einem oder mehreren der Ansprüche 1 bis 3, dadurch gekennzeichnet, daß zur Sicherstellung oder zur Wiederherstellung einem freien Durchganges der Meßleitung (14, 15, 17) bzw. zur Vermeidung einer Verstopfung, Abknickung oder Verschluß der Meßleitung (14, 15, 17) eine Gasspülungsvorrichtung für die Druckmeßleitung (14, 15, 17) vorgesehen ist.

5. Vorrichtung nach Anspruch 1, dadurch gekennzeichnet, daß die Druckdifferenzmessung $\Delta P$ zwischen Tubusende (12) und Tubusanfang (11) ein Signal bei Verstopfung, Abknickung oder Verschluß des Tubus (5) erzeugt und daß der Tubus (5) mit einer Absaugvorrichtung zur Störungsbeseitigung verbunden ist.

6. Vorrichtung nach einem oder mehreren der vorhergehenden Ansprüche, dadurch gekennzeichnet, daß zur Kontrolle der Intratrachealdruckmessung $P_T$ oder anstelle dieser Messung in die Speiseröhre (18) eine ballonartige Meßvorrichtung (20) (z.B. Ösophagussonde) einführbar ist, die ein Anliegen der Speiseröhre (18) an der elastischen Trachealwand (19) bewirkt und daß Druckschwankungen in der Trachealröhre (4) zu einer Ausdehnung oder Kompression der Trachea (4) führen, die von der Meßvorrichtung (20) erfaßbar sind.

**Claims**

1. A device for automatically regulating the spontaneous respiration assistance at the respirator of a resuscitation machine, with an endotracheal tube (5) with, at the input end and constructed as a Y-piece (7), an adaptor for the connection of an inhalation (21) or exhalation line (22) to a respirator, the tube (5) extending into the bottom half of the trachea (4), an airway pressure-measuring facility being provided in the inhalation phase to regulate the supply of gas from the respirator and whereby, furthermore, to compensate for the friction pressure losses of the gas in the tube (5) an over-pressure $P_Y$ created by the respirator is generated at the Y-piece (7), characterized in that for automatic compensation of the friction pressure loss of the endotracheal tube (5) ends in the lower tracheal area (26) a pressure-measuring line (14, 15, 17) which extends outwardly and serves to measure the intratracheal airway pressure $P_T$, with a pressure-measuring instrument (16), and in that the necessary. over-pressure $P_Y$ at the Y-piece (7) can be so adjusted by means of the respirator as a function of the gas flow created by the patient during his spontaneous respiration or by the desired or necessary pulmonary pressure level that the airway pressure $P_T$ desired at the tube end (12), i.e. at the intratracheal pressure-measuring point is adjusted, whereby in the absence of spontaneous respiration by the patient, no over-pressure is created in the Y-piece and in that in the tube intake zone (11) or in the respirator there is a pressure-measuring device (13) for measuring the airway pressure $P_Y$ there and which is connected by a differential circuit to the tracheal pressure-measuring device (16) and in that the pressure difference $\Delta P = P_Y - P_T$ or $P_T - P_Y$ defined during the individual respiration and resuscitation phases can be ascertained, whereby malfunctions triggered by blockage, kinking or occlusion can be detected.

2. A device according to Claim 1, characterized in that the airway pressure measured at the tube end (12) triggers the delivery of gas via a trigger means, e.g. threshold value circuit.

3. A device according to claim 1 or 2, characterised in that airway pressure measurement triggers delivery of gas via the regulator, said delivery being adapted to the needs of the patient.

4. A device according to one or more of Claims 1 to 3, characterised in that to ensure or restore a free passage through the measuring line (14, 15, 17) and/or to avoid blockage, kinking or occlusion of the measuring line (14, 15, 17) a gas scavenging device is provided for the pressure-measuring line (14, 15, 17).

5. A device according to Claim 1, characterised in that the pressure difference measurement ΔP between tube end (12) and tube commencement (11) creates a signal in the event of blockage, kinking or occlusion of the tube (5) and in that the tube (5) is connected to a vacuum extraction device to remedy faults.

6. A device according to one or more of the preceding Claims, characterised in that for monitoring the intratracheal pressure measurement $P_T$ or instead of this measurement it is possible to introduce into the feed pipe (18) a balloon-like measuring device (20), e.g. oesophageal probe, which causes the feed pipe (18) to rest on the flexible tracheal wall and in that fluctuations in pressure in the tracheal tube (4) lead to an expansion or compression of the trachea (4) which can be detected by the measuring device (20).

**Revendications**

1. Dispositif pour le réglage automatique de l'assistance à la respiration spontanée dans un respirateur d'une machine respiratoire, comportant un tube endotrachéal (5), comportant, du côté d'entrée, une pièce d'adaptation, réalisée sous forme de pièce en Y (7), pour raccorder un conduit d'inspiration (21) ou un conduit d'expiration (22) à un respirateur, le tube (5) parvenant dans la moitié inférieure de la trachée (4) et, dans la phase d'inspiration, une mesure de la pression de voie respiratoire pour régler l'alimentation en gaz du respirateur étant prévue et, par ailleurs, pour compenser les pertes de pression de friction du gaz dans le tube (5), il est engendré une surpression $P_Y$, créée par le respirateur, sur la pièce en Y (7), caractérisé en ce que, pour la compensation automatique de la perte de pression de friction du tube endotrachéal (5), un conduit de mesure de pression (14,15,17) s'achève dans la zone inférieure de la trachée (26), conduit qui est guidé vers l'extérieur et sert a mesurer la pression de voie respiratoire intratrachéale $P_T$ par un appareil de mesure de pression (16), en ce que la surpression nécessaire $P_Y$ sur la pièce en Y (7) peut être réglée, de façon dépendant du flux de gaz engendré par le patient lors de sa respiration spontanée ou du niveau de pression des poumons nécessaire ou souhaité, au moyen du respirateur, de sorte que la pression de voie respiratoire $P_T$ souhaitée à l'extrémité du tube (12), c'est-à-dire à la position de mesure de pression intrachéale, est réglée, aucune surpression n'étant engendrée dans la pièce en Y lorsque la respiration spontanée du patient fait défaut, en ce que, dans la zone de l'entrée du tube (11) ou dans le respirateur, il est prévu un dispositif de mesure de pression (13) de la pression de voie respiratoire locale $P_Y$, qui est relié, par l'intermédiaire d'un circuit différenciateur, au dispositif de mesure de pression trachéale (16), et en ce que la différence de pression $\Delta P = P_Y - P_T$ ou $P_T - P_Y$, définie pendant les phases respiratoires individuelles, peut être détectée, des fonctionnements défectueux déclenchés par un bouchage, un flambage ou une obturation pouvant être détectés.

2. Dispositif selon la revendication 1, caractérisé en ce que la pression de voie respiratoire mesurée à l'extrémité du tube (12) déclenche l'alimentation en gaz par l'intermédiaire d'un dispositif déclencheur, par exemple un circuit à seuil.

3. Dispositif selon la revendication 1 ou 2, caractérisé en ce que la mesure de pression de voie respiratoire déclenche une alimentation en gaz adaptée au patient par l'intermédiaire du respirateur.

4. Dispositif selon une ou plusieurs des revendication 1 à 3, caractérisé en ce que, pour garantir ou rétablir un passage libre du conduit de mesure (14, 15, 17) ou pour empêcher un bouchage, un flambage ou une obturation du conduit de mesure (14,15,17), il est prévu un dispositif de nettoyage au gaz pour le conduit de mesure de pression (14,15,17).

5. Dispositif selon la revendication 1, caractérisé en ce que la mesure de différence de pression $\Delta P$ entre l'extrémité du tube (12) et l'entrée du tube (11) engendre un signal lors du bouchage, du flambage ou l'obturation du tube (5), et en ce que le tube (5) est relié à un dispositif d'aspiration pour éviter ces problèmes.

6. Dispositif selon une ou plusieurs des revendications précédentes, caractérisé en ce que, pour le contrôle de la mesure de pression intratrachéale $P_T$ ou à la place de cette mesure, un dispositif de mesure du type ballonnet (20), par exemple une sonde de l'oesophage, peut être introduit dans le tube digestif (18), dispositif qui fait s'appuyer le tube digestif (18) contre la paroi élastique de la trachée (19), et en ce que des variations de pression dans la trachée (4) entraînent une dilatation et une compression de la trachée (4), qui sont détectables par le dispositif de mesure (20).

Fig 1

Fig 2

Fig 3-8 = Stand der Technik

Fig 3

Fig 4

Fig 5

Fig 6

Fig 7a

Fig 7b

Fig 7c

DRUCKVERLUST DER ATEMWEGE
mit bzw. ohne TUBUS

Flow L/min
■ Ohne TUBUS  + mit TUBUS

Fig 8